Europäisches Patentamt

(19) European Patent Office

Office européen des brevets

(11) Publication number: **0 328 123**
**A1**

## (12) EUROPEAN PATENT APPLICATION

(21) Application number: **89102269.1**

(22) Date of filing: **09.02.89**

(51) Int. Cl.⁴ **A61K 39/29 , A61K 39/40 , C12N 15/00**

(30) Priority: **09.02.88 US 154678**

(43) Date of publication of application:
**16.08.89 Bulletin 89/33**

(84) Designated Contracting States:
**AT BE CH DE ES FR GB GR IT LI LU NL SE**

(71) Applicant: **Eugene Tech International, Inc.
No. 4 Pearl Court
Allendale New Jersey 07401(US)**

(72) Inventor: **Youn, B. Woo
340 Island Avenue
Ramsey New Jersey 07446(US)**
Inventor: **Samanta, Himadri K.
28 Lincoln Place
Waldwick New Jersey 07463(US)**

(74) Representative: **Vossius & Partner
Siebertstrasse 4 P.O. Box 86 07 67
D-8000 München 86(DE)**

(54) Heterogeneous, pre-S rich hepatitis B surface antigen.

(57) This invention relates to heterogeneous, pre-S rich hepatitis B surface antigen (HBsAg) particles purified from mammalian cell culture medium, and to a method of purification that avoids harsh treatment. The HBsAg particles of the invention contain substantial amounts of both S, both pre-S1, and both pre-S2 components, and the particles can be used to produce a potent vaccine against type B hepatitis infections. The invention also relates to a transfected mammalian cell culture containing an expression vector and capable of producing the heterogeneous, pre-S rich particles of the invention.

EP 0 328 123 A1

EP 0 328 123 A1

## HETEROGENEOUS, PRE-S RICH HEPATITIS B SURFACE ANTIGEN

This invention relates to heterogeneous, pre-S rich hepatitis B surface antigen (HBsAg) particles purified from culture medium, to a method of purification, and to an expression system. The HBsAg particles of the invention contain substantial amounts of pre-S1, pre-S2, and S components and the particles can be used commercially to produce a potent vaccine against type B hepatitis infections.

Hepatitis B virus (HBV) infects hundreds of millions of people annually. Hepatitis B virus is an etiologic agent of serum or transfusion hepatitis and is associated with the development of chronic liver disease and primary hepatocellular carcinoma.

Subunits of the hepatitis B virus envelope proteins have been employed as antigens to induce protective immune response against infections. For instance, surface protein components of the HBV virus, HBsAg, have been purified from viral Dane particles obtained from the plasma of persons chronically infected with hepatitis B virus, and made into a vaccine. The surface protein components are referred to as HBV surface antigen, or HBsAg, because of their ability to evoke an immune response.

It it is inadvisable to use a human carrier's serum as a source of HBsAg, because the serum may contain the active, highly infectious hepatitis B virus, as well as undesirable adventitious agents. There are limited numbers of donors and limited quantities of plasma-derived antigen available for purification, and plasma-derived vaccines require labor-intensive and costly purification procedures, such as Vnek et al., U.S. 3,951,937, and Ohmura et al., U.S. 4,565,697, precluding their use in developing countries where hepatitis presents a major health problem. Furthermore, purification of a plasma-derived vaccine, such as those of Blumberg et. al., U.S. 3,636,191, and Hillman et al. 1983, Viral Hepatitis: Standardization in Immunooroohvlaxis of Infectious by Hepatitis Viruses, pp 3-12, often requires treatment with substances such as pepsin, urea, or formalin to remove dangerous impurities, and such treatment reduces the activity of the vaccine.

Hepatitis vaccines produced by recombinant DNA (rDNA) technology and vaccines produced in cell culture avoid the problems associated with vaccines derived from human plasma.

Three classes of organisms have been used to express proteins using rDNA techniques: the prokaryotic organism, E. coli; the unicellular eukaryote, yeast; and mammalian cells in continuous culture. Expression levels in E. coli are low, and the immunogenicity of the product is minimal. The particles produced by yeast are not secreted and have low antigenicity. Cultured mammalian cells provide the most natural and preferred system for expression of viral antigens in vaccine production, because viruses depend entirely upon the host cell's biosynthetic machinery for functions such as protein synthesis, proper folding, processing, glycosylation, assembly into multimers, and secretion from the cell.

The synthesis and secretion of components of HBsAg from mammalian cells in culture is known. Fitts et al., U.S. 4,696,898. See Davis et al., 1985. Proc. Natl. Acad. Sci. USA 82, 7560-7564; Denniston et al., 1984 Gene 32, 357-368; Dejean et. al., 1983 Proc. Natl. Acad. Sc:. USA 80, 2505-2509; and Patzer et al., 1985 Vaccine 85, pp. 261-264. The HBsAg particles synthesized in and secreted from mammalian cells in culture medium are morphologically, biochemically and immunologically similar to plasma-derived HBsAg particles. HBsAg particles secreted from mammalian cell culture may contain the same protein components as those in plasma, including S and some of the pre-S proteins. Cell culture-derived HBsAg is somewhat immunogenic, is less expensive than plasma-derived vaccine, and lacks many of the perceived risks of plasma-derived vaccine. However, these cell culture-derived HBsAg vaccines lack some of the pre-S components found in native HBV.

Hepatitis B virus has one of the smallest DNA sequences known for mammalian viruses, around 3200 bp. The HBV DNA has an unusual structure -- circular and partially double stranded. The length of the double stranded region is variable. It is also believed that a protein is covalently linked to the 5' end of the long (L) strand. The polymerase associated with this virion can repair the single stranded region of the viral genome in an in vitro reaction to make it fully double stranded DNA. Sequencing of HBV DNA has revealed that there is substantial heterogeneity in sequences in genomes from HBV of different subtypes.

Tiollais et al., "The Hepatitis B Virus", Nature 317: 489-495 (1985) describe the structure of HBV and its pre-S1, pre-S2 ahd S gene products.

Murray, U.S. 4,710,463 describes recombinant DNA molecules comprising an HBV DNA sequence capable of expressing HBV surface antigen in a unicellular host such as E. Coli.

Kim et al., "Cloning and Expression of Hepatitis 3 Surface Antigen gene," Korean Biochem. J. 17: 70-79 (1984) disclose pBR322 plasmids containing HBV DNA, as does Ono et al., Nucleic Acid Res. 11: 1747 (1983).

Pavlakis et al., "Applications of Bovine Papilloma Viral and Retroviral Vectors," Cold Spring Harbor, pp. 29-38 (1987) discuss vectors for expression of virus DNA in mammalian host cells.

2

Millich et al., U.S. 4,683,136, obtained HBsAg from chinese hamster ovary cell culture. The HBsAg contained one of the pre-S2 region components according to gel electrophoresis and that region was found to be more immunogenic than S region antigen.

Levinson et al., European Patent Application 0073656, published March 9, 1983 discloses vectors producing an expression HBsAg particles including essentially only an S region component.

The prior art references do not disclose an expression system capable of expressing heterogeneous, pre-S rich HBsAg particles in mammalian cell culture.

Hershberg, U.S. 4,624,918, teaches a method for purifying HBsAg from recombinant cell culture using ion exchange chromatography with or without immunoaffinity adsorption (immunoadsorption) chromatography. Ion exchange chromatography separates the HBsAg into fractions according to the heterogeneous charge distribution of the HBsAg particles, and therefore may reduce the heterogeneity of the HBsAg particle preparation.

Immunoadsorption chromatography is also inadvisable. It is expensive because it requires that monoclonal antibody be produced on a large-scale, purified, and coupled to the immunoaffinity column gel media. Immunoadsorption chromatography may also contaminate the sample due to leaching out of mouse monoclonal antibody, another foreign protein that has to be removed in downstream purification steps.

Kawahara et al., U.S. 4,515,714 teaches an affinity chromatography step using sulfated gel media. Sugahara et al., U.S. 4,612,283 teaches that yeast-derived recombinant HBsAg particles may be purified using hydroxylapatite column chromatography but the HBsAg final product was not heterogeneous, and showed only a single 24Kd band in SDS-polyacrylamide gel electrophoresis. Affinity chromatography, like ion exchange chromatography, can be expected to reduce the heterogeneity of an HBsAg preparation.

Ultracentrifugation has been used to purify spherical HBsAg particles approximately 22 nm in diameter with a density of approximately 1.2 g/l. Ultracentrifugation is generally employed after initial concentration and fractional precipitation, such as in Wampler, U.S. 4,349,539, or by affinity, ion exchange, or hydroxylapatite column chromatography, and several successive ultracentrifugations are generally required to isolate HBsAg particles with a desirable purity. See Gerin et al. J. Virol. 7:569 (1971) and J. Immunol. 115:100 (1975). Repeated ultracentrifugation reduces the yield of HBsAg.

It is an object of this invention to obtain a mammaliah cell culture that expresses a complete HBsAg gene to produce pre-S rich HBsAg particles.

It is also an object of this invention to isolate from mammalian cell culture HBsAg particles rich in pre-S components and with heterocenecus charge by avoiding harsh treatment and separation techniques that reduce the heterogeneity of HBsAg particles.

It is therefore an object of the invention to produce pure heterogeneous HBsAg particles in high yield without using hydroxylapatite, or immunoadsorption chromatography and without using proteolytic, acidic or other aggressive treatments.

The present invention relates to a heterogeneous hepatitis B surface antigen (HBsAg) and a process for purifying the heterogeneous HBsAg from mammalian cell culture medium.

The process of the present invention starts with a suitable mammalian cell line that secretes heterogeneous, pre-S rich HBsAg particles into the culture medium.

The preferred expression system is a mammalian cell culture transfected with a vector containing and capable of expressing the entire hepatitis B surface antigen gene, including the pre-S1 and pre-S2 regions. Cell lines such as CV-1, CHO, 3T3, and HeLa may be used. The most preferred expression system comprises mouse fibroblast cells transformed with a vector that includes genetic material from bovine papilloma virus, a mouse metallothionein gene promoter, a human metallothionein gene and a hepatitis B virus surface antigen gene, such that the promoter inducibly controls the expression of the HBsAg gene product, which is capable of formation into pre-S rich heterogeneous HBsAg particles.

A recombinant DNA expression vector according to the invention comprises a gene determinant for HBsAg comprising pre-S1, pre-S2 and S region DNA from HBV and an inducible control region, inducible by an inducer, a gene conferring antiotic rsistance to a bacterial host, optionally a transformer gene capable of transforming a mammalian cell line, and a gene conferring selective advantage on transfected cells relative to untransfected cells in the presence of a selecting agent, the HBsAg gene being positioned relative tc the control region such that it is subjected to the control of the control region, whereby upon introduction of the vector into a bacterial host, the vector can be cloned by selection with the antibiotic, reproduced, and multiple copies can be obtained, and whereby upon introduction of the vector into a mammalian cell line host, transfected cells can be selected in the presence of the selective agent and the control region is able to allow production of pre-S rich HBsAg to proceed in response to presence of the inducer, and the mammalian cell line host is capable of secreting heterogeneous, pre-S rich HBsAg particles.

In accordance with the purification process of the invention, first, a crude preparation of HBsAg is isolated. The cell culture medium, which may contain up to about 25%, preferably about 10% added serum, is clarified and concentrated. Preferably, cell debris is removed by medium speed centrifugation, and the supernatant is concentrated about 10-50 fold by ultrafiltration, in order to reduce the volume without adversely increasing viscosity. Concentration may be accomplished by ultrafiltration, most preferably using a filter with a molecular weight cut-off somewhat less than the molecular weight of the HBsAg particles. Thus, a pore size of less than 1 million is preferred. A cut-off about 300,000 is most preferred. Protein concentration preferably does not exceed about 200 mg/ml.

Second, HBsAg particles are fractionally precipitated from the concentrate, preferably using. plythylene glyccl. Preferably, tne precipitare containing HBsAg particles is pelleted by medium-speed centrifugation. This step produces a crude preparation which is then purified in the subsequent steps.

Third, the HBsAg pellet is dissolved in a minimum volume of a buffer and applied to a gel filtration chromatography column, and fractions containing HBsAg are collected.

Fourth, the HBsAg fractions are subjected to isopycnic banding ultracentrifugation, preferably using potassium bromide (KBr) as a density medium in a concentration about 24%-26%. The fractions containing HBsAg are collected and subjected to concentration-dialysis.

Fifth, the HBsAg containing fractions are subjected to a sucrose step gradient ultracentrifugation, preferably using 20%, 30% and 40% (w/w) sucrose, and the fractions containing pure HBsAg particles are collected and either dialyzed or subjected to a buffer exchange using a suitable gel filtration column chromatography medium.

The resulting HBsAg is pure and suitable for lyophilizing and for preparing a safe and effective vaccine against hepatitis. The inventive process is simple, economical and appropriate for large-scale industrial use. The vaccine has beneficial antigenic properties due to an abundance of highly immunogenic pre-S components and can be used by delivery to the bloodstream of a patient suffering from hepatitis.

The HBsAg product of the invention is heterogeneous and consists essentially of two forms of S protein, two forms of pre-S2 protein, and two forms of pre-S1 protein in amounts producing upon SDS-PAGE and silver-staining of the gel, a percentage stain density of about 70%-90% S protein, about 5%-20% pre-S2 protein and about 2%-10% pre-S1 protein.

The figures show:

Fig. 1 is a flow chart for a process of purifying HBsAg from recombinant cell culture.

Fig. 2 is a silver-stained sodium dodecyl sulfate-polyacrylamide gel electrophoresis pattern for purified HBsAg.

Fig. 3 is a flow chart for cloning the HBV genome in pBR322 to produce pHB1.

Fig. 4 is a flow chart for cloning the S-gene of HBV into the expression vector pCS1 to produce pTHB1.

Fig. 5 is a flow chart for constructing the expression clone pHPS1.

Fig. 6 is a flow chart for constructing pHPS3.

Fig. 7 is a flow chart for constructing pHPS5.

Fig. 8 is a flow chart for constructing pHPS7.

Fig. 9 is a flow chart for constructing pHPS11.

Fig. 10 shows details of vector pCS1.

Fig. 11 shows details of vector pCS2.

Fig. 12 shows details of vector pCS3.

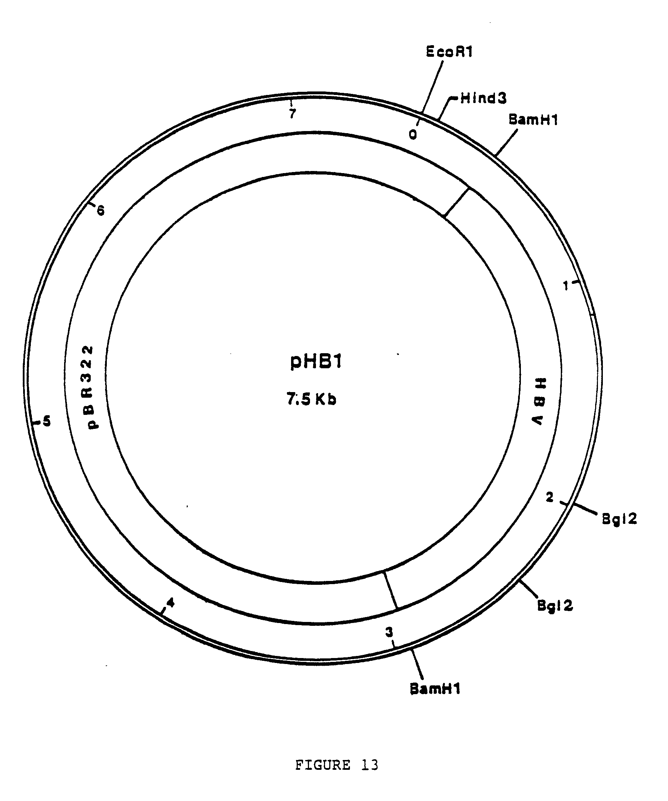

Fig. 13 shows details of vector pHB1.

Fig. 14 shows details of vector pTHB1.

Fig. 15 shows details of vector pHPS1.

Fig. 16 shows details of vector pHPS3.

Fig. 17 shows details of vector pHPS5.

Fig. 18 shows details of vector pHPS7.

Fig. 19 shows details of vector pHPS11.

The starting material for the present invention is a mammalian cell line that expresses the hepatitis B virus gene. The cell line may be genetically engineered, viral infected, or derived from an infected patient. The preferred starting material for the invention is HBsAg produced using a recombinant virus vector as a vehicle and mammalian tissue culture cell line as an expression host.

Vectors according to the invention: (1) express the entire HBsAg gene, including pre-SI, pre-S2, and S regions; (2) can propagate both in bacteria and mammalian host cells; (3) have at least one marker for selecting bacteria after transformation; (4) have at least one marker for selecting mammalian cells after

4

transfection; (5) can exist in multiple copies in the host cell to produce a high transcription level; and (6) have a strong promoter to drive the transcription of HBV surface antigen gene. Most preferably, if the promoter has to be induced, the inducer is inexpensive and appropriate for mass production of HBsAg from the producing cell line.

A preferred vector includes: (1) a portion of pBR322 providing an origin of replication in E. coli and an ampicillin resistance gene, (2) bovine papilloma virus DNA for propagation in mouse cell lines in multiple copies; (3) a mouse metallothionein promoter as a strong promoter for transcription of HBsAg gene; and (4) a human metallothionein gene as a selectable marker for mouse cells in the presence of cadmium. Both metallothionein promoters can be induced by cadmium or zinc, which are inexpensive. The human metallothionein gene has an additional benefit of causing the host cell to increase copies of the plasmid in presence of cadmium, thus amplifying expression.

Purifying HBsAg from a mammalian cell culture medium is quite different from purifying HBsAg from human blood. First, the mammalian cell culture medium contains foreign proteins including as much as 10% or more fetal bovine serum and host cell secretion products. In order to use recombinant HBsAg as a vaccine for human injection, the final product must be free of any foreign proteins contained in the starting material.

Second, the concentration of HBsAg produced in culture medium is much lower than that contained in high-titer human carrier plasma. As a consequence, although the protein concentration and diversity is much higher in human blood than in culture medium, the amount of contaminants per unit of HBsAg is much larger in culture medium than in human blood.

Another difficulty in purifying HBsAg particles from culture medium arises from the heterogeneous charge and density distributions of HBsAg particles due to the abundance of pre-S1 and pre-S2 components in them. The biochemical and biophysical heterogeneity of HBsAg depends on the gene construction and gene expression systems used in culture.

Native HBsAg Dane particles contain three peptide chains: S protein is a 226 amino acid protein with an unglycosylated form, molecular weight 24,000 daltons (p 24) and a glycosylated form, molecular weight 27,000 daltons (gp 27). Pre-S2 protein is a 281 amino acid protein present in two glycosylated forms with molecular weights of 33-34,000 (gp 34) and 36-37,000 daltons (gp 36), whose amino acid sequence contains the S region together with a 55 amino acid pre-S2 region at the amino-terminal end. Pre-S-1 protein includes about 400 animo acids and is present in two glycosylated forms with molecular weights about 41,000 (gp 41) and about 44.000 (gp 44). Pre-S1 protein contains the S region, the pre-S2 region, and a pre-S1 region. The 226 amino acid S region is largely conserved. The 55 amino acid pre-S2 region has more variability among HBV subtypes. The pre-S1 region is most variable, and may even have different lengths, ranging from about 108 to about 128 amino acids. See Tiollais et al., "The hepatitis B virus," Nature 317: 489-495 (1985).

The aggregated quaternary structure of native HBsAg particles is believed to include about 90% S protein, 8-9% pre-S1 protein, and 1-2% pre-S2 protein together with lipids in a roughly 22 nm spherical shape. The amino acid composition and degree of glycosylation of the proteins varies depending on virus subtype, and the relative amounts of each protein in the quaternary, multimeric structure of the HBsAg particle may vary, too.

In accordance with the present invention, it is desirable to preserve essentially all pre-S components during purification because these components are thought to be important in inducing protective immunity against HBV infection. The starting HBsAg contains a substantial amount of pre-S1 and pre-S2 components in addition to the major S antigens. Heterogeneous HBsAg particles produced in accordance with the invention are roughly spherical and have a mean diameter about 22 nm, and are morphologically and immunologically similar to HBsAg particles isolated from carrier human plasma.

Although the precise biochemical and biophysical properties of the 22 nm particle are not completely known, the present inventors have found that HBsAg particles produced according to the invention have a wide range of charge and density distributions, probably due to post-translational modifications, carbohydrate heterogeneity and complex formation of the pre-S1, pre-S2, and S gene products. In accordance with the present invention, all variants of form expressed and secreted into the culture medium are desirably included in the final product, heterogeneous, pre-S rich HBsAg particles.

According to the present invention, heterogeneous pre-S rich HBsAg particles are obtained by using techniques that separate particles on the basis of physical characteristics of size and density while avoiding techniques that separate components on the basis of chemical characteristics such as charge and immunoaffinity or other chemical affinity.

The present inventors have also observed that repeated ultracentrifugations using cesium chloride might remove most of the pre-S components from the HBsAg particles, presumably due to a high salt effect. It is advantageous to preserve the pre-S components because they are believed to be important for the full immunologic potency of an HBsAg vaccine, and so the process of purifying HBsAg from cultured HBsAg particles in the present invention avoids the use of excessive ultracentrifugations.

## Purification Process

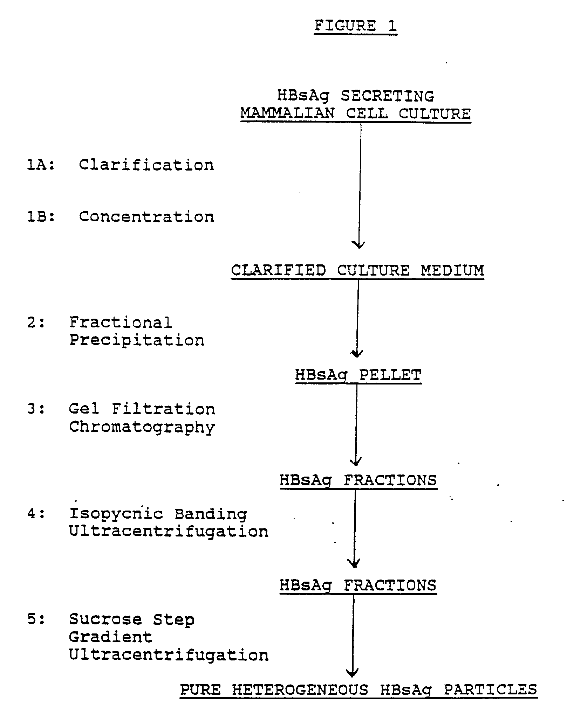

Referring to the flow diagram in Fig. 1, in the first step, the collected cell culture medium is clarified by centrifuging at medium speed, preferably for 10-20 min. at 10,000 G, to remove the host cell debris. The present inventors found that before this clarification step, the culture medium may be stored at 2-8~C for an extended period of time without significant deterioration in HBsAg activity. No protease activity was found in the culture medium using a commercially available protease tablet assay such as that developed by BioRad.

The clarified fraction is then concentrated, preferably by ultrafiltration at 2-8~C, and when serum is present, the molecular weight cut-off for the filter should be about 300,000 daltons. A tangential Megaflow system manufactured by New Brunswick Scientific employing an XM-300 membrane filter manufactured by Amicon, is a suitable system for concentrating a large quantity of culture medium while maximizing HBsAg yield. The average HBsAg yield is more than 95% in this step. Filters that have a cut-off above about 1 million are considered microfiltration and will lead to some loss in yield.

When the cell culture medium contains 10% serum, the medium should be concentrated about 10-fold. When the cell culture medium has no added serum, it is preferable to concentrate the medium to more than 50-fold. With no added serum, the preferable molecular weight cut-off for the ultrafiltration membrane may be less than when serum is present. The protein concentration should not exceed about 200mg/ml because the concentrate would become too viscous for uniform mixing of polyethylene glycol in the next step.

In the second step, HBsAg is recovered in a fractional precipitation procedure, preferably in one step. The preferred precipitating agent is polyethylene glycol (PEG), having a molecular weight of 6,000-9,000 daltons. A particularly effective precipitation is accomplished by adding PEG powder slowly with stirring at room temperature for about 30 min., and leaving the resulting admixture for more than 3 hrs., preferaoly overnight, at 2-8~C. The object of the one step precipitation is to enrich HBsAg with minimal contaminating proteins, so it is desirable to obtain a concentration of PEG, just less than the amount that precipitates HBsAg almost completely. A concentration of 9-12% (w/w) is preferred, most preferably 10%, with an HBsAg yield of 80-90%. Employing more than 12% PEG gives an HBsAg yield of greater than 90% but also precipitates contaminating proteins which are very difficult to remove in later steps.

Other protein precipitating agents such as, e.g., ammonium sulfate may be utilized in place of PEG as long as they accomplish the same objectives described above. Adding 50% ammonium sulfate precipitates more than 80% of HBsAg from the culture medium. However, this procedure appears to co-precipitate more impurities, especially bovine serum albumin, than the 10% PEG precipitation procedure described above. The precipitated HBsAg is recovered by medium-speed centrifugation, suitably performed at 10,000 G for 30 min.

In the third step, the precipitate is dissolved in a minimum volume of buffer for gel filtration chromatography. The preferred technique uses 2-3 ml phosphate buffered saline for the precipitate obtained from every 50 ml of 10X concentrated culture medium. Undissolved material is removed in a clarification step, suitably performed by centrifugation at 20,000 G for 10 min.

The sample solution is then subjected to gel filtration column chromatography in order to enrich HBsAg, including the pre-S components, while removing contaminating proteins which might cause a protein over-loading effect in the subsequent isopycnic banding step. Gel filtration has been used as a buffer-exchange step and to remove some impurities at the end of the purification process. Hershberg, U.S. 4,624,918. According to the present invention, heterogeneous HBsAg particles containing pre-S components can be enriched with a high yield using gel filtration column chromatography prior to ultracentrifugation, rather than using ion exchange, affinity or hydroxylapatite column chromatography. Gel filtration is also preferable to ultrafiltration for removing protein contaminants because an ultrafiltration filter retains contaminants such as bovine serum albumin, probably as complexes.

An appropriate buffer can be chosen for gel filtration, thus obviating the need of buffer-exchange or concentration-dialysis in subsequent ultracentrifugation steps, and reducing the number of repeat ultracentrifugations necessary. The gel filtration medium should be resistant to packing in tall columns used in large-scale preparation, and should separate the high molecular weight HBsAg particles from lower molecular weight contaminating components such as bovine serum albumin. The chromatography medium is able to separate HBsAg from other low and high molecular weight molecules and has a desirable column separation range in the high molecular weight region in a given length of a column such as from about 1,200,000 to 100,000 daltons in molecular weight.

A suitable range allows particles to elute partially in the vbid volume and partially in the inclusion volume, so that the upper range of the exclusion limit is about the molecular weight of the HBsAg particle, preferably about 1-2 million. The lower range should be high enough to permit effective separation of the HBsAq particles from other components in a convenient length of column, preferably about 100,000 daltons. A suitable medium is ultrogel AcA22, available from LKB, a gel comprising 2% agarose and 2% acrylamide.

The gel filtration step separates highly enriched HBsAg fractions in a single peak from high and low molecular weight proteins. Because of the low amount of contamination, HBsAg can be separated as a single peak with high purity and yield when subjected to the KBr isopycnic banding in the next step.

A suitable amount of chromatography medium, such as ultrogel AcA22, is approximately 1L of resin for a starting quantity of 5 L of culture medium before ultrafiltration, containing about 50 mg of HBsAg. The column is equilibrated, for example, with PBS, and gel filtration chromatography is performed at either 2-8~C or room temperature.

Fractions from the gel filtration column chromatography are then examined by SDS-PAGE for the presence of HBsAg under reducing conditions. Visualization of the SDS-PAGE is achieved by silver staining as developed by Merril, Science 211, 1437-1438 (1981). A mini-gel electrophoresis system with a gel thickness of less than 0.5 mm can obtain high resolution in a short time, even less than 2-4 hours. HBsAg-containing fractions are collected according to the results of the silver-stained gel pattern. It is preferable to pool only those fractions which are low in high molecular weight proteins and bovine serum-derived proteins. The overall yield after step 3 is about 70-80%, the single-step yield is more than 85%.

Thereafter, the HBsAg particles are separated on the basis of density by ultracentrifugation, preferably isopycnic ultracentrifugation followed by sucrose step gradient ultracentrifugation. Thus, in step 4, the pooled HBsAg-containing sample may be further purified to a near homogeneity by isopycnic ultracentrifugation.

Isopycnic ultracentrifugation separates the HBsAg particles in a broad peak, based on their density range. Higher density contaminants such as bovine serum albumin, and lower density contaminants such as cell culture products are separated into separate peaks. Suitable materials include cesium salts of bromide or chloride, and potassium salts of bromide or tartarate. KBr performs better than CsCl in providing maximum yield and high purity. KBr has a density range from about 1.1 - 1.4, providing a shallower gradient than CsCl, which has a density range from about 1.1 - 1.7. KBr- banded HBsAg fractions appear to contain less impurities than CsCl-banded sample. Moreover, KBr is more cost-effective than CsCl.

In the preferred embodiment, the pooled fractions containing HBsAg collected from the gel filtration column chromatography are dialysed, and solid KBr is added. The preferred concentration of KBr is 24-26% (w/w). The sample solution is divided into appropriate tubes and centrifuged to equilibrium, e.g. at 150,000 G for 20-24 hrs. at 10~-15~C. Suitable changes that have to be made according to the process scale in terms of the choice of centrifuge tube, rotor size, or rpm will be apparent to workers in the field.

The linear density gradient formed as a result of isopycnic ultracentrifugation is fractionated using an appropriate gradient fractionation system, and the fractions are identified by SDS-PAGE and silver staining techniques. The purity of the HBsAg product after the step 4 should be more than 85%.

Analysis of the purified fraction by SDS-PAGE reveals the presence of S, pre-S1 and pre-S2 antigens. Other minor contaminating proteins can also be detected, such as high molecular weight proteins and bovine serum albumin. If the contaminating proteins are found in excess of 20% of the total protein, however, it is preferable to carry out another ultracentrifugation run after readjusting the KBr concentration at 24-26% (w/w) in the pooled fractions from the first ultracentrifugation.

The pooled fractions are dialysed or diafiltrated and concentrated at the same time with PBS using a filter with molecular weight cut off about 300,000 daltons, such as an Amicon membrane filter XM-300. Diafiltration and concentration are performed at 4~C by concentrating the sample solution to a minimum volume, then adding a desired buffer to be dialyzed, preferably PBS, in order to remove KBr, because KBr interferes with sucrose density gradient ultracentrifugation. The solution is concentrated again to a minimum volume. This cycle is repeated about 3-5 times to ensure complete removal of KBr.

Depending on the process scale, the product concentration can be very high. Preferred techniques in this regard are to dialyze the sample solution at 4~C against phosphate bufferred saline (PBS), or to desalt it by gel filtration column chromatography using appropriate column materials, having a low exclusion range, preferably about 30,000 or less, as is commonly known in the art. Sephadex G-25 or G-50 from Pharmacia can be used.

In the fifth step, the dialyzed sample solution is subjected to sucrose step gradient ultracentrifugation. A suitable sucrose step gradient according to the present invention is composed of 20%, 30% and 40% (w/w). However, addition of a 10% sucrose gradient step may be required if the sample contains a large amount of impurities after the KBr ultracentrifugation in step 4. The sample solution is overlayered and centrifuged at 4~C for instance, at 150,000 G for 16-18 hr. The type of rotor and length of centrifugation time to be employed should be selected depending on the size of the HBsAg sample and the concentration of HBsAg in the sample solution, which also depends on the process scale. Such variables will be apparent to those skilled in the art.

After ultracentrifugation, the gradient is fractionated using a gradient fractionation system. The optical density profile monitored at 280 nm during the gradient fractionation shows a single peak containing HBsAg particles preceded by a minor peak containing mostly the contaminating proteins. SDS-PAGE results indicate that these are mostly the high molecular weight proteins, greater than 66,000 daltons. The purity of the HBsAg product (including all the pre-S components) contained in the single peak is in excess of 98% and is essentially free of other proteins and DNA, as measured by currently available techniques. The overall yield after step 6 is about 30-40%.

The pooled fractions containing the purified product are then dialyzed against an appropriate buffer and may be used to produce a vaccine.

HBsAg Product

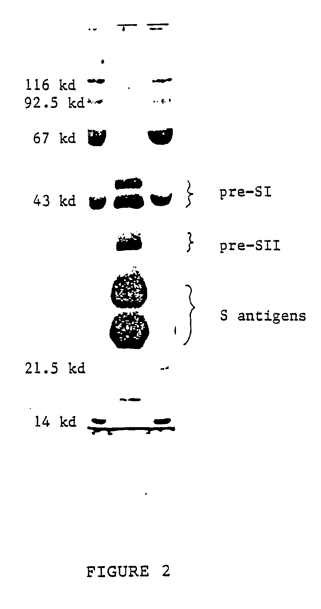

Figure 2 shows SDS-PAGE analysis of the final HBsAg product obtained from a 5L starring culture. The silver stained gel pattern indicates tne presence of proteins having molecular weights of approximately 24, 27, 34, 37, 41 and 44 kilodaltons, corresponding to the S, pre-S2, and pre-S1 gene products. Immunological identification of the gene products is made with an immunoblot staining technique using various monoclonal and polyclonal antibodies.

After SDS-PAGE and either coomassie blue R-250 or silver staining, gel scanning densitometry analysis demonstrates that the product is at least 98% pure. The product of the invention can be consistently satisfactorily identified by the relative stain intensity of the 6 components. The relative stain intensity of S protein is about 70%-90%, of pre-S2 protein about 5%-20%, and of pre-S1 about 2%-10%. The relative stain intensity of the pre-S2 and pre-S1 components after silver staining is higher than the expected staichiometric percentage. Reported figures for pre-S2 are about 8%; stain intensity is about 5%-20%. Reported figures for pre-S1 are about 1%; stain intensity is about 2-10%. Without limiting the scope of the invention, this discrepancy can possibly be explained as due to a selective concentration of the pre-S components during purification, but more likely the glycosylated pre-S components selectively stain more intensely than the S components in the silver-staining technique employed according to the invention.

When the final HBsAg product is stored at 4~C in PBS (pH 7.4), the pre-S components are shown to undergo a rapid degradation while the S gene product remains intact. More rapid degradation of the pre-S components is observed to occur at either slightly alkaline (pH 8.5, 50 mM Tris-HCL buffer) or acidic (pH 5.0, PBS) condition. The present inventors have found that the best way to preserve the pre-b components is to lyophilize the product and store it at 4~C. No significant decrease in stability of the pre-S product is observed even after one year.

It is estimated that the amount of DNA present (if any) is less than 6 picograms per milligram heterogeneous, pre-S rich HBsAg produced by the 5 L process described in example 2. This value is the limit of detection determined by direct measurement of DNA in the final product by dot-blot hybridization technique.

To make the purified heterogeneous HBsAg into a vaccine, the product may be first subjected to a two-step heat-inactivation and potentiation process, e.g. according to Prince, U.S. 4,639,371. The first step consists of heating the final product solution at 102~C for 2 minutes and 40 seconds under pressure. This flash-heating step is carried out for HBsAg immunopotentiation and virus inactivation. The flash-heated sample is further heated at 65~C for 10 hr. Finally, the heat-treated HBsAg is compounded with an adjuvant such as aluminum phosphate in the form of a gel. As a result of these treatments, a highly efficacious vaccine may be made. Each dose of adjuvant-vaccine contains 3 micrograms HBsAg and 0.01% (w/v) thimerosal.

Expression Vectors for Mammalian Cell Culture

In accordance with the invention it is possible to achieve active and stable expression of an HBsAg gene to produce heterogeneous, pre-S rich HBsAg particles. Apart from the promoter strength and the copy number of the gene, the expression can also depend on the presence or absence of an enhancer, the size of 5' untranslated region and the size of 3' untranslared region. According to the invention, one puts into the expression vector as little DNA as possible without sacrificing the expression level, in order to minimize unnecessary information in the expression system and also to make the DNA easier to handle.

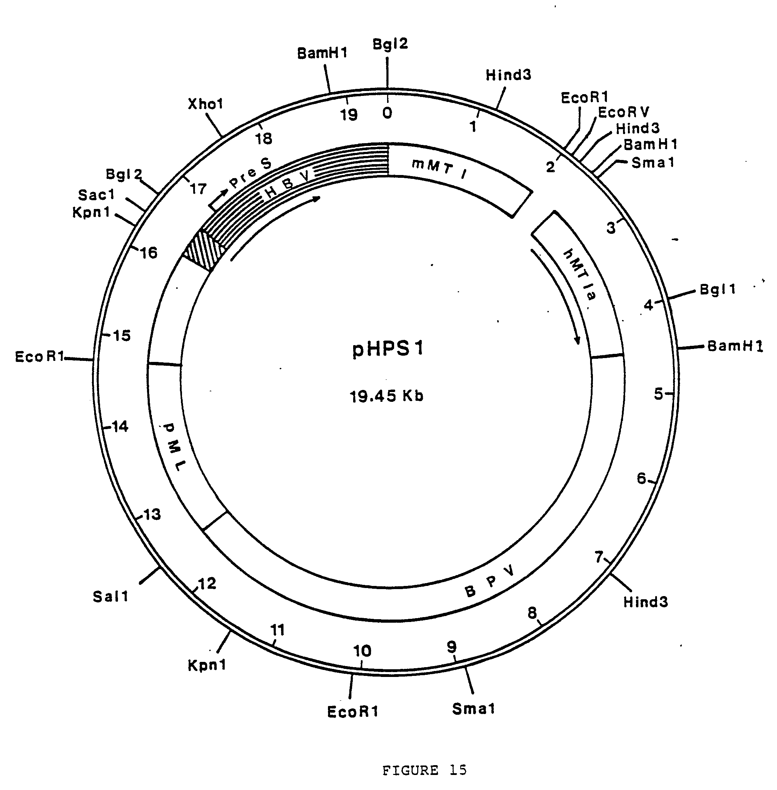

In accordance with the invention, different constructions can be made and used to transfect or transform a mammalian cell culture, such as mouse C127 cells. Stable transformants can be cloned, and the production of surface antigen from each clone can be assayed by both radioimmunoassay (RIA) and enzyme-linked immuno-sandwich assay (ELISA). A preferred constructs according to the invention is pHPS1 and related constructs pHPS3, pHPS5, pHPS7 and pHPS11 are shown in Figures 15-19. These plasmids may be obtained by methods known in the art.

In summary, pHPS1 has a full length BPV genome, complete but interrupted mouse metallothionein (mMT) gene and 86% of the HBV DNA. The polyadenylation for the HBsAg site is provided by the HBV genome. Figure 15. The whole mouse metallothionein gene without any promoter is also present in the construct.

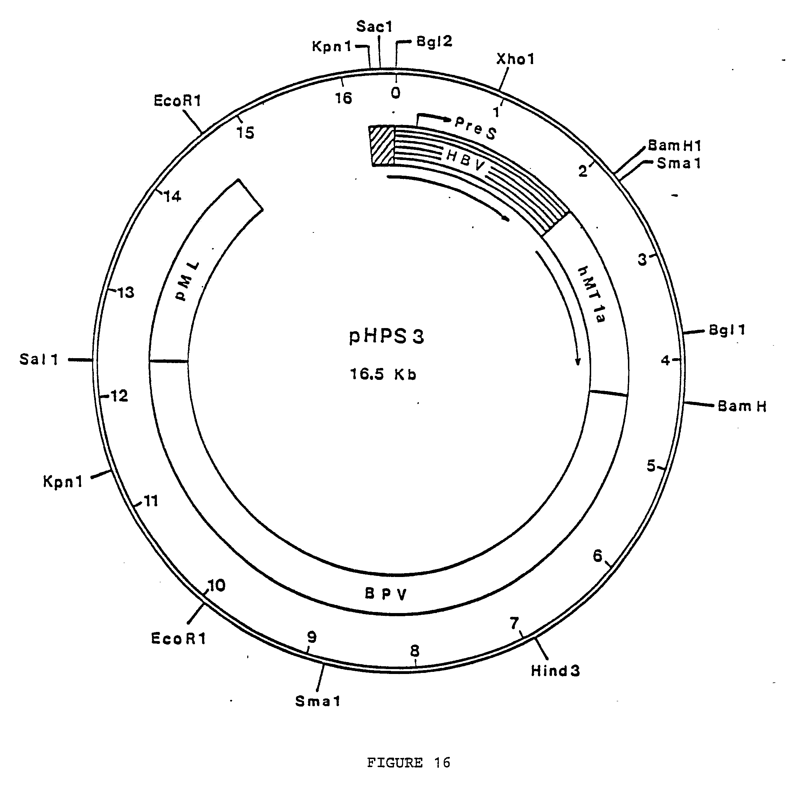

pHPS3 is a derivative of pHPS1 from which most of the 3' untranslated region, the polyadenylation site of HBV DNA, and extra mMT DNA has been removed. Figure 16.

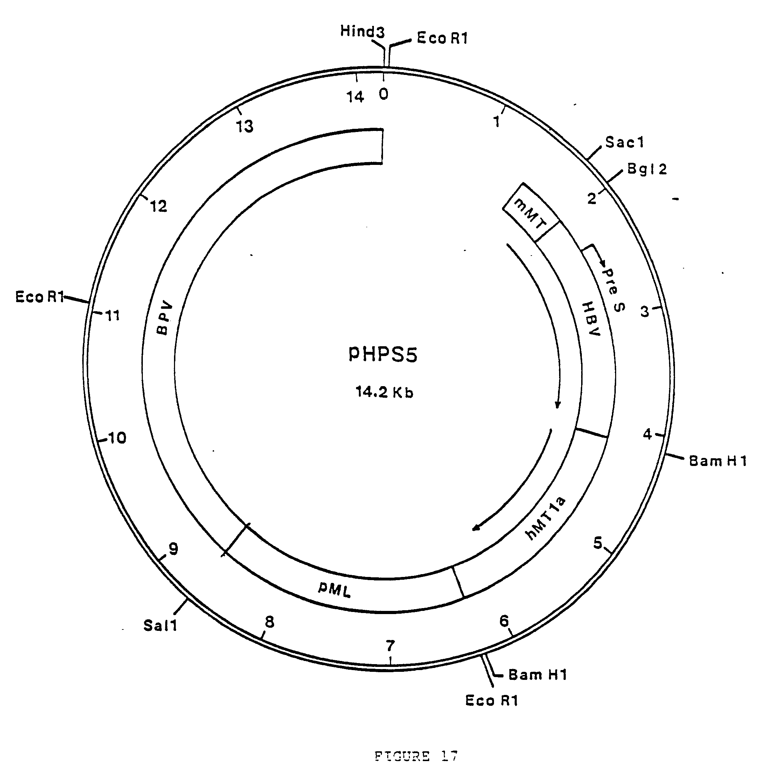

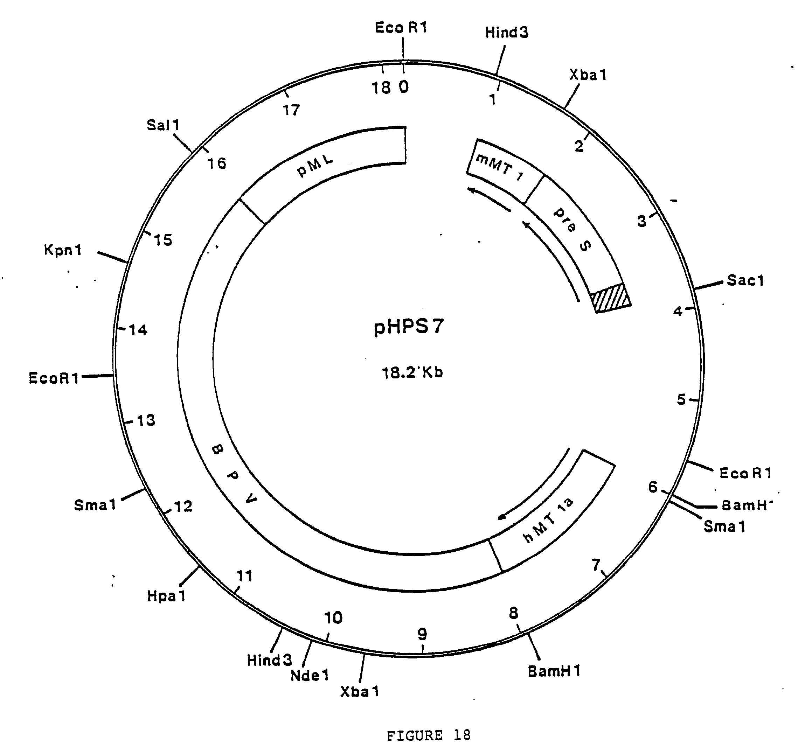

pHPS5 is an even shorter version of pHPS3, with only 69% of BPV DNA. Figure 17. In pHPS7, the 5' untranslated region is removed so that transcription starts just in front of the beginning of the pre-S coding region. Figure 18.

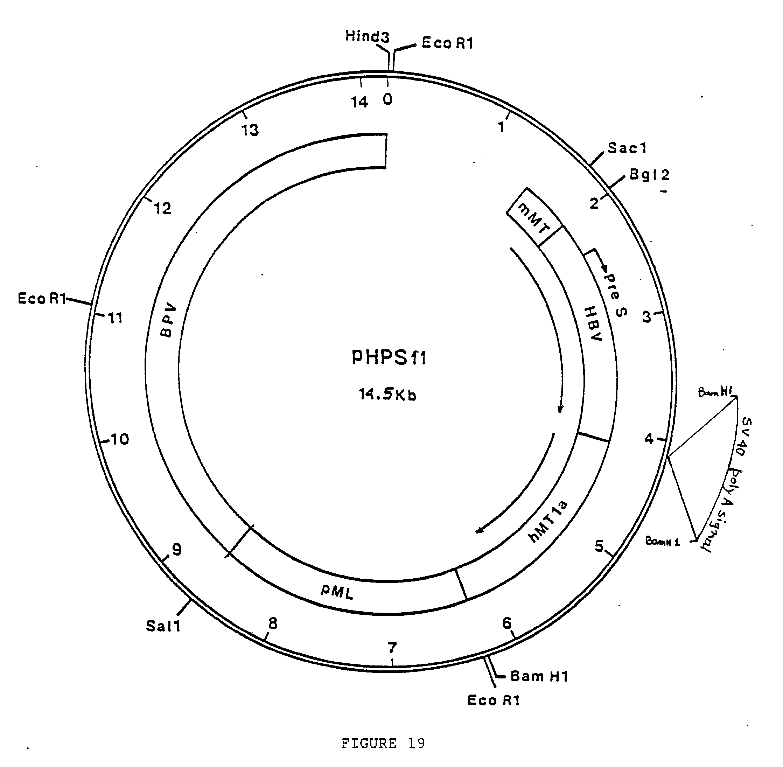

pHPS11 is the same as pHPS5, except that the polyadenylation signal from SV40 DNA is located just after the surface antigen coding region in pHPS11. Figure 19.

In all the above constructs except pHPS7, the transcription starts from a considerable distance (450 bases) from the beginning of the coding region of the surface antigen.

A vector of the invention is capable of being selected and replicated in bacterial culture so that substantial amounts of the vector can be obtained and used to transfect a mammalian cell culture.

According to the invention, a vector capable of expressing an HBV S gene is used to transfect a mammalian cell culture and the cells are induced to transcribe and translate pre-S1, pre-S2 and S gene products, to make post-translational modifications such as glycosylation, to assemble the polypeptides into particles, and to secrete pre-S rich, heterogeneous HBsAg particles. The cells may also produce and secrete unassembled polypeptide gene products of the S gene, including pre-S regions, so long as the heterogeneous HBsAg particles of the invention are produced.

The critical effect of transfecting a mammalian cell culture according to the invention is to make the cell culture capable of secreting pre-S rich, heterogeneous HBsAg particles. The cells may be transformed with a transformer gene, which may allow for a high rate of production of HBsAg particles and a high cell density, but in accordance with the invention, any mammalian cell culture can be employed as host, and any vector as transfecting agent so long as the cells are thereby enabled to secrete heterogeneous pre-S rich HBsAg particles.

The details of the construction of pHPS1, pHPS3, pHPS5, pHPS7, and pHPS11 are given in examples 1 to 6. A method of transfecting mammalian cell culture cells is given in example 7. A method of purifying the product of the invention is described in example 8.

The invention is illustrated by the following examples, but is not limited to them.


## EXAMPLE 1


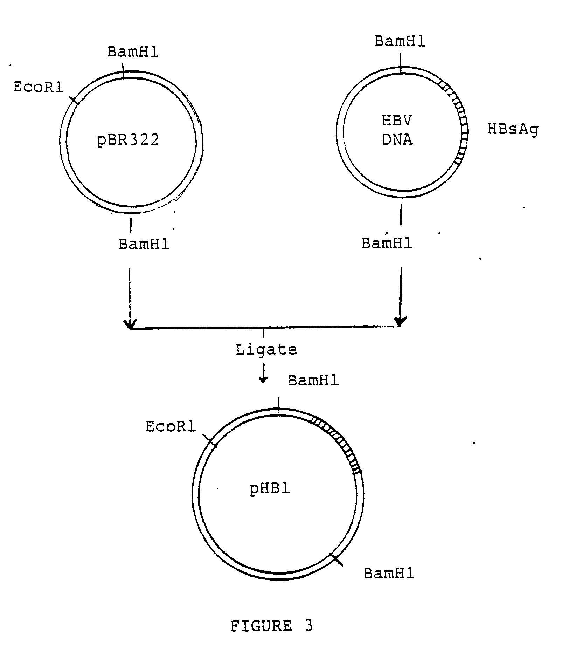

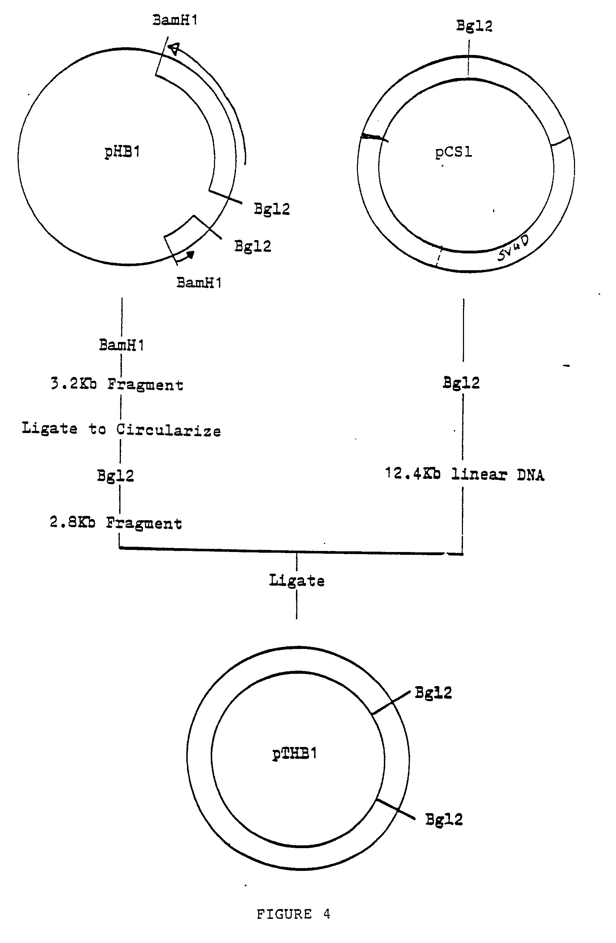

### Construction of pTHB1 (see figures 3 and 4):

Hepatitis B virus Dane particles were obtained from the serum of an adr-subtype HBV carrier, and incubated with polymerase to make a complete double stranded DNA sequence. The DNA was isolated, digested with BamH1, and cloned in a pBR322 plasmid at the BamH1 restriction site to produce plasmid pHB1. Figure 3.

As shown in Figure 4, plasmid pTHB1 was constructed by digesting 20 micrograms of pHB1 with BamH1 and purifying the 3.2 Kilobase HBV-specific DNA fragment on an agarose gel. The HBV DNA was recircularized with the help of T4 ligase, then digested by BglII to produce a 2.8 kilobase fragment and a smaller fragment. The larger 2.8Kb BglII fragment was purified on sea plaque agarose gel. This fragment was then inserted with the help of T4 ligase at the BglII site of plasmid pCS1 vector DNA.

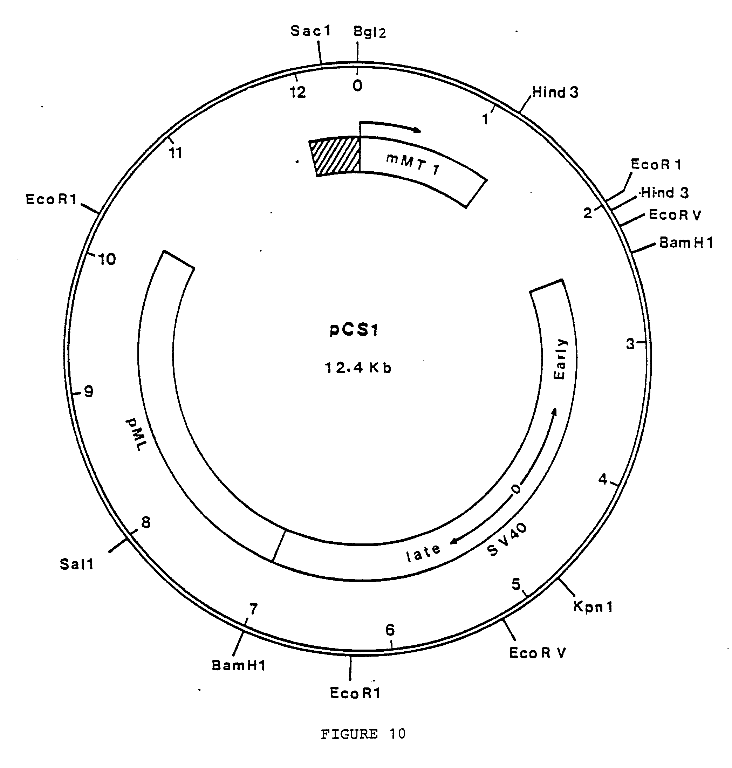

Plasmid pCS1 includes an SV40 gene and a mouse metallothionein gene (mMT) in a pML vector derived from pBR322. See Figure 10. The BglII site of pCS1 is downstream from the mouse metallothionein (mMT) promoter and upstream from the AUG start codon of the MT gene, so that the expression of the HBV fragment came under the control of the metallothionein gene.

After ligation the reaction product was ethanol precipitated and the DNA was taken up in TE medium (10mM Tris pH 8.0., 1mM EDTA).

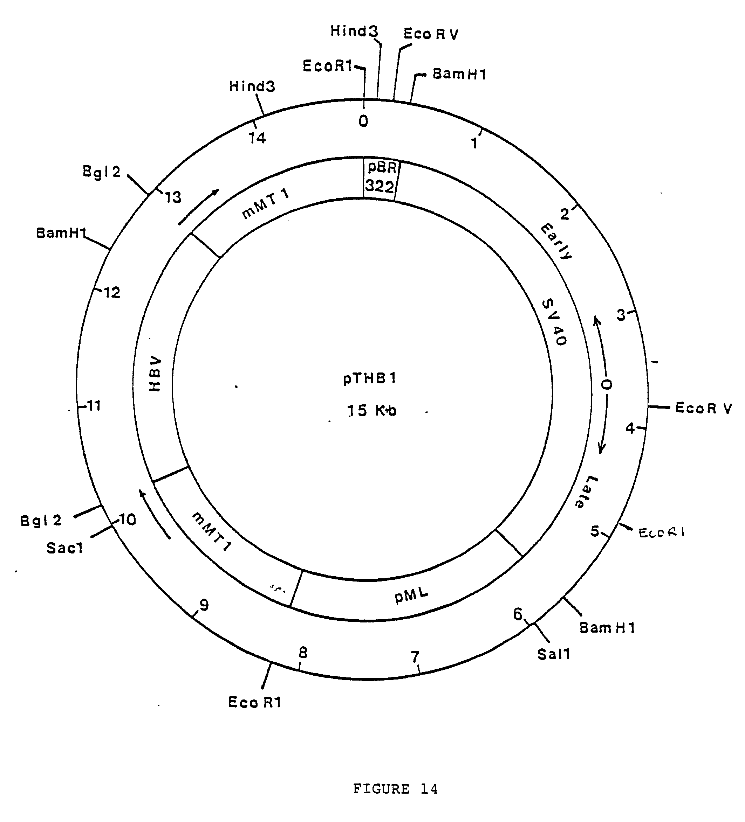

The pTHB1 DNA was then used to transform competent HB101 bacterial cells and the transformants were selected on an ampicillin plate. Individual colonies were picked up and grown vernight at 37~ C. A mini-plasmid preparation was done for ach colony and the DNA was digested with different enzymes to heck the insert size and the orientation of the insert. One colony with correct orientation was picked-up and the clone was named pTHB1. The structure of pTHBI is shown in Figure 14.

Clone pTHB1 was grown, plasmid DNA was purified on a esium chloride gradient and was used to transform a CV-1 cell line. Two days after transformation the supernatant was ssayed for HBsAg secreted by the cells into the medium by ELISA. We detected the presence of surface antigen at a low level.

## EXAMPLE 2

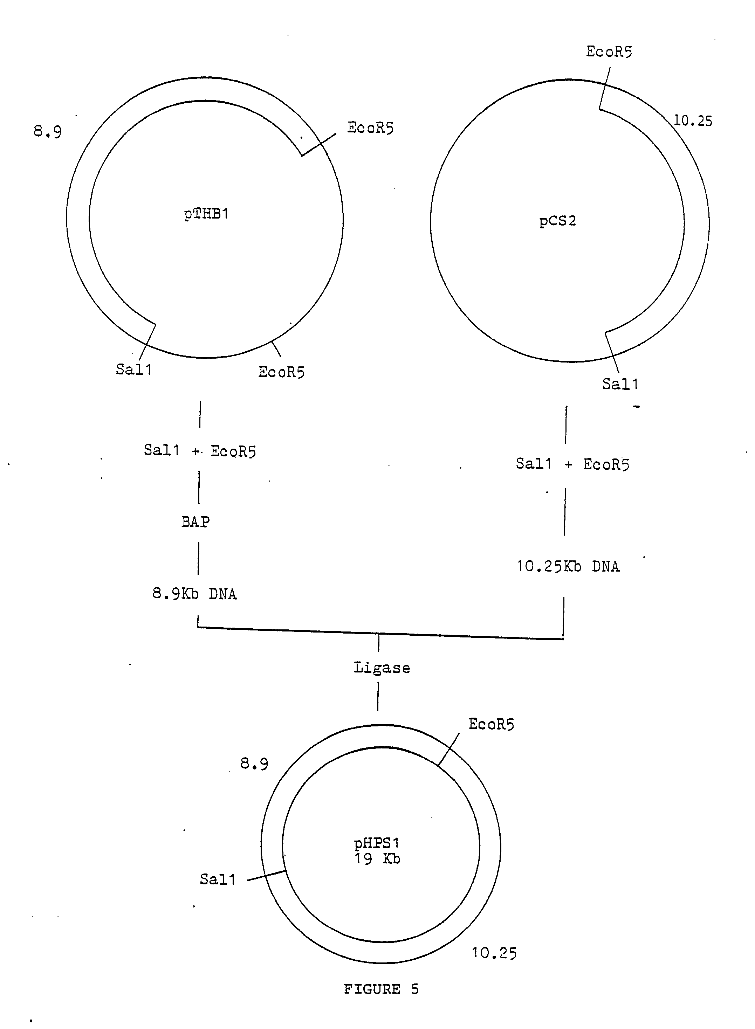

### Construction of pHPS1: (see figures 5 and 15)

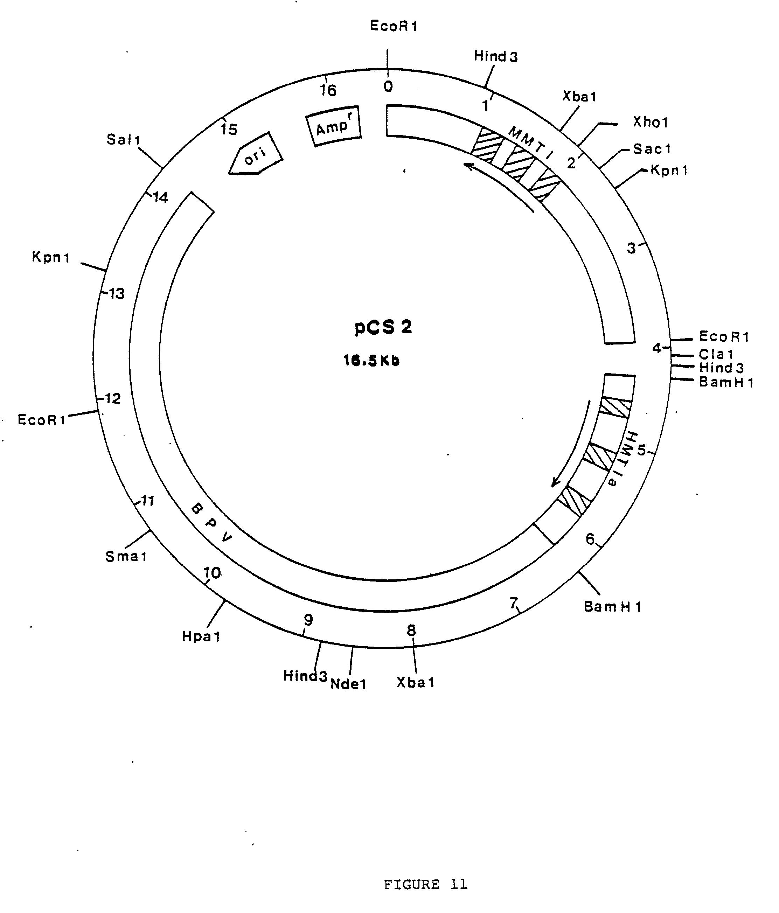

pTHB1 was digested with two restriction enzymes, EcoR5 and SalI, dephosphorylated by incubating with bacterial alkaline phosphatase and the resulting 8.9 kb fragment was purified on a sea plaque agarose. This fragment was ligated with a 10.25 kb fragment generated by digesting pCS2 with EcoRV and SalI. pCS2 is shown in Figure 11. After transforming HB101, mini plasmid preparations were made and plasmids were analyzed by digesting with different enzymes.

## EXAMPLE 3

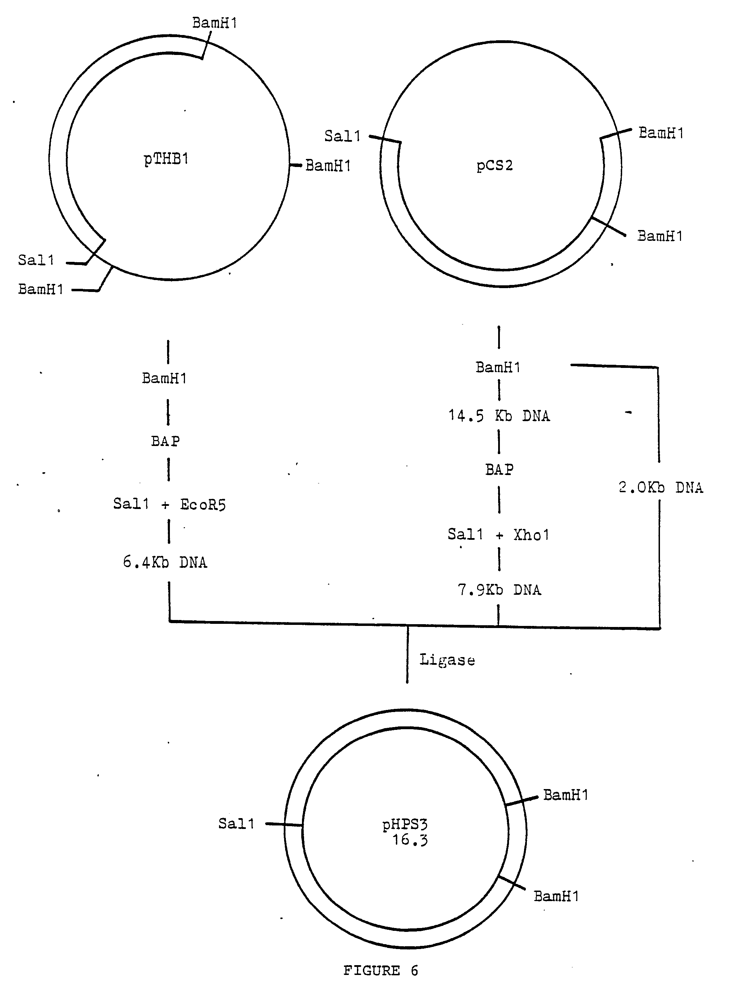

### Preparation of pHPS3 (see Figures 6 and 16)

pTHB1 was digested with BamH1 and bacterial alkaline phosphatase (BAP). After phenol extraction and ethanol precipitations DNA was further digested by SalI and EcoR5 and the 6.4kb SalI/BamHI fragment was purified on sea plaque agarose. Plasmid pCS2 (Figure 11) was also digested with BamH1, and then run on an agarose gel to purify a 2.0 kb human metallothionein (HuMT) gene and a 14.5kb fragment. The latter was digested with BAP, phenol extracted and ethanol precipitated followed by a digestion with SalI and XhoI. The resulting 7.9kb SalI/BamH1 fragment was purified, and mixed with the 2.0kb HuMT fragment and the 6.4kb fragment from pTHB1. The fragments were ligated and the plasmid was used to transform HB101 cells.

Mini plasmid preparations were made and restriction enzymes used to check the orientation and copy number of different fragments in the plasmid. One of those with correct orientation and copy number was picked up and called pHPS3. In this procedure, some of the dephosphorylations were done only on one end of the fragments to increase the chance of ligation with proper orientation and to increase the chance of forming multimers.

## EXAMPLE 4

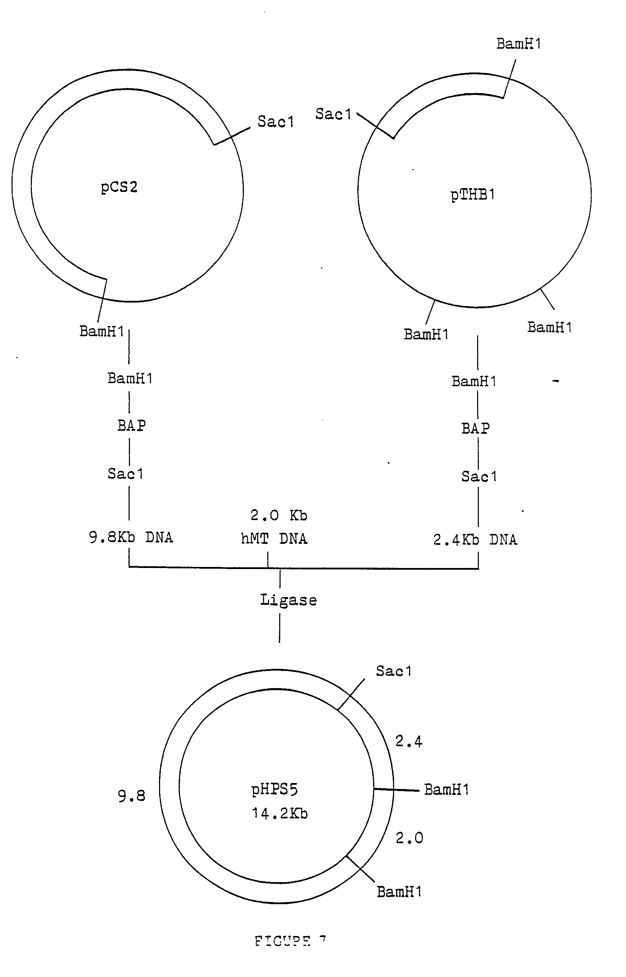

### Construction of pHPS5 (see Figures 7 and 17)

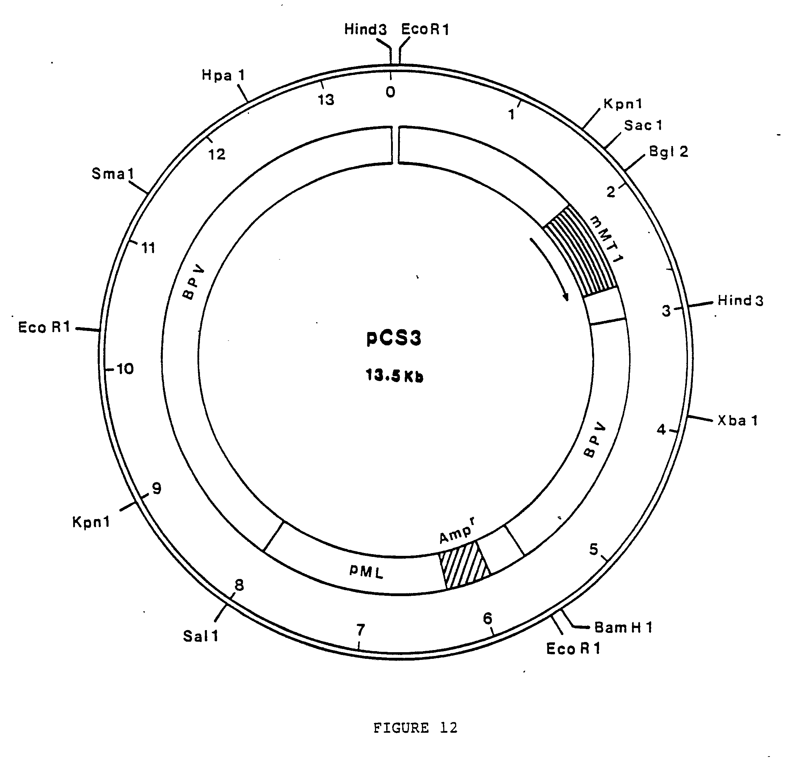

Both pTHB1 and pCS3 (Figure 12) were digested separately with BamH1, BAP and then with SacI. The 9.8kb SacI/BamHI fragment of pCS3 and the 2.4kb BamHI/SacI fragment of pTHB1 were purified on agarose gel. The 2kb HuMT fragment was also purified as described in example 3. All these 3 fragments were mixed and ligated as in example 3. The plasmid with correct orientation and copy number of fragments was selected and called pHPS5.

EXAMPLE 5

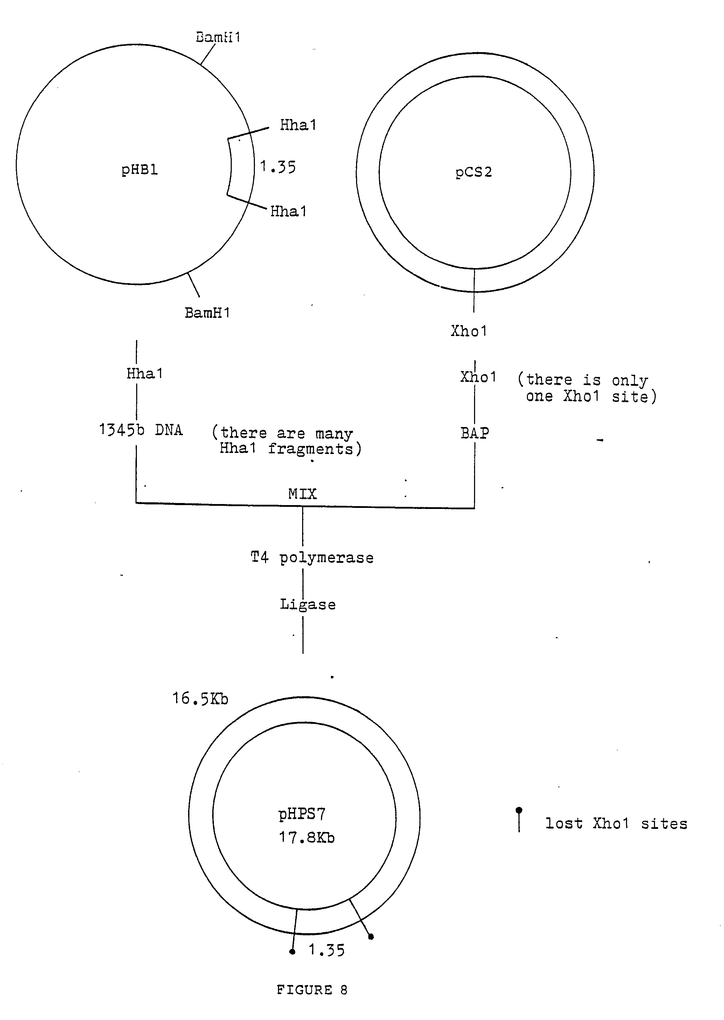

Construction of pHPS7 (see Figures 8 and 18)

pHB1 was digested with BglII and Hhal and run on agarose gel and the largest band, 1.35kb, was purified. pCS2 (Figure 11) was also digested at the Xhol site and dephosphorylated. Equimolar amounts of the Hhal fragment and the Xhol digested pCS2 were mixed and incubated for 30 min. at 37~ C with T4 polymerase in the presence of all four deoxyribose nucleotides. Hhal produces 3~ overhang and Xhol produces 5~ overhang, and incubation with T4 polymerase made both kinds of ends blunt. The sample was then heated at 65~ C for 10 min., extracted with phenol, ethanol precipitated with ethanol, and taken up in ligation buffer for overnight ligation.

Using procedures known in the art, HB101 was transformed with the ligated material. Individual colonies were picked up, mini-plasmid preparations were made and restriction enzymes used to identify a plasmid with single insert in correct orientation. That plasmid was selected and called pHPS7.

EXAMPLE 6

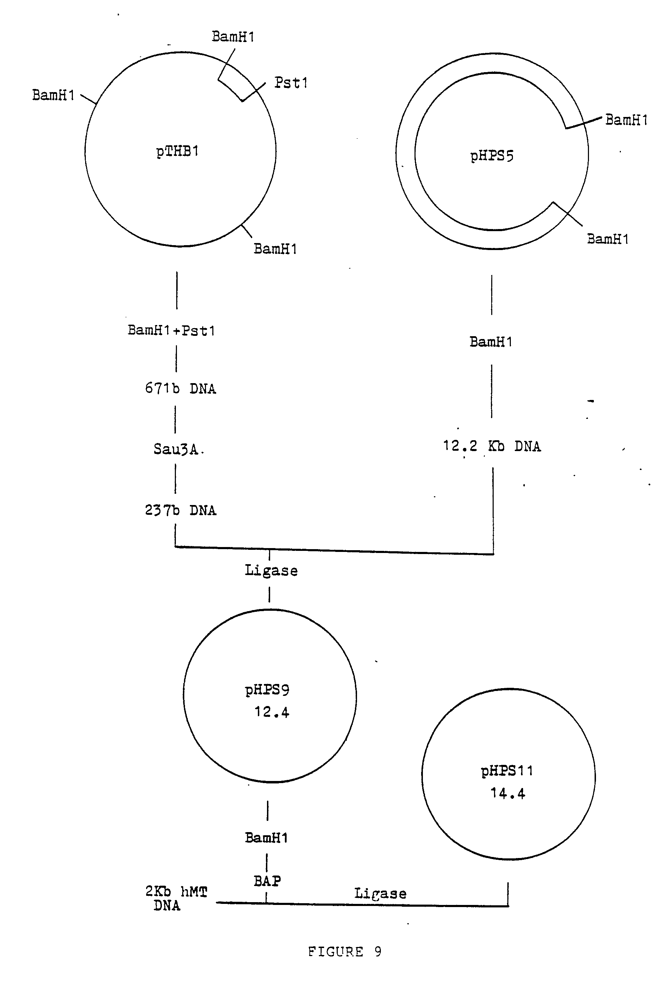

Construction of pHPS11 (see Figures 9 and 19)

pTHB1 was digested with BamH1 and Pst1 and 617bp DNA was purified. This fragment was digested with Sau3A to produce a 237 bp Sau3A/BamH1 SV40 DNA fragment. A 12.2kb BamH1/BamH1 fragment of pHPS5 was prepared and ligated to the 237bp fragment. The correctly oriented plasmid was isolated and called pHPS9. The BamH1 end and Sau3A and ligated with each other be:ause they have complementary protruding ends but both sites got lost at that junction. The other junction (BamH1/BamH1 ends) however remains digestable with BamH1. That site was again opened up with BamH1 and the 2kb BamH1/BamH1 HuMT fragment from pCS2 (Figure 11, Example 3) was put there. The final plasmid is called pHPS11.

EXAMPLE 7

Constructs pHPS1, pHPS3, pHPS5, pHPS7, and pHPS11 were used to transfect mouse monolayer C127 fibroblast cells and were challenged with 20 micromolar cadmium ions, Cd + +. Normally Cd + + is toxic to the challenged cells, unless the cells are able to produce excess metallothionein because they have been tranfected by the construct which can produce metallothionein. Metallothionein binds with Cd + +, giving the transfected cell a selective advantage in presence of Cd + +, so that only the cells which have picked up the vector constructs containing MT gene will survive. The presence of Cd + + also induces the HBV surface antigen gene which, in this construct, is under control of a metallothionein promoter.

Mouse C127 cells were grown in monolayer in 10 cm dishes in Dulbecco minimal essential medium available from GIBCO as DMEM with 10% fetal bovine serum added. 30 microgram of plasmid DNA, twice purified on CsCl gradient was used per dish. Calcium phosphate precipitation technique was used to transfect cells. Four hours after transfection the medium was exchanged with fresh medium.

The next day the cells were split in the ratio of 1:3 and after they became attached to the plate the media was replaced with fresh media with 10 uM of CdC12 in it. Many cells died during the next 3 to 4 days and every 3 to 4 days growth media was exchanged, keeping cells under the challenge of cadmium. After 2 to 3 such changes cadmium concentration was increased to 20 micromolar. After 2 to 3 weeks, visible colonies. resistant to 20 micrcmolar Cd + - were formed.

They were then cloned individually and grown separately in small petri dishes. Media from each clone was assayed for HBsAg using an RIA kit available from Abbott. For each construct, four to five clones were selected, and the amount of HBsAg released per $10^6$ cells per 24 hours was determined for each clone. The results are shown in Table 1.

It is apparent from Table 1 that plasmid HPS1 produced the most HBsAg. Plasmid pHPS11 was also productive, while plasmids pHPS3, pHPS5, and pHPS7 were only slightly productive.

Table 1

| Expression of HBsAg by various vector constructs. | | | | | |
|---|---|---|---|---|---|
| Expression vector | Expression of HBsAg (ng/106 cells/24hr.) | | | | |
| | Clone 1 | Clone 2 | Clone 3 | Clone 4 | Clone 5 |
| pHPS1 | 1100 | 900 | 1900 | 2100 | - |
| pHPS2 | 44 | 60 | 14 | 42 | 36 |
| pHPS3 | 20 | 16 | 11 | 12 | - |
| pHPS4 | 44 | 36 | 27 | 34 | 44 |
| pHPS5 | 427 | 159 | 241 | 188 | 197 |

EXAMPLE 8

Purification of Heterogeneous, pre-S Rich HBsAg

This example illustrates the purification of heterogeneous, pre-S rich HBsAg from cell culture medium by the procedure shown in Figure 1. The culture medium contained 10% fetal bovine serum and was obtained from cell cultures of the mouse epithelial cells of Example 7 in roller bottles. The expression vector was pHPS1.

Five liters of culture medium as in Examples 2 and 7 was centrifuged at 10,000 G for 30 min. to remove cells and cell debris. Clarified culture medium was then concentrated 10 fold at 4~C by ultrafiltration using a Megaflow filtration system (New Brunswick Scientific) with an Amicon membrane filter XM-300, whose molecular weight cut-off value is about 300,000 daltons. After the retentate volume reached about 300-400 ml, the system was flushed once with 100-200 ml of distilled water for an effective recovery of HBsAg.

The controlled culture medium was subjected to one-step fractional precipitation by 10% polythylene glycol of molecular weight 6000-9000 (PEG 6000-9000). Precipitation was initiated at room temperature by slowly adding 50g of powdered PEG to 500g of 10-fold concentrated culture medium, and then carried out overnight at 4~C. The precipitate formed was then spun down at 10,000 G for 30 min., and the pellet was dissolved in about 25 ml of PBS. After a brief clarification centrifugation at 20,000 G for 10 min. to remove any undissolved material, the crude sample solution was applied to a Ultrogel AcA22 column (5 cm in diameter and 50 cm in height) pre-equilibrated with PBS.

After the sample was loaded, the column was run at 3-4 cm/hrs. at room temperature. A typical elution profile showed that HBsAg elutes as a partially resolved first peak on the leading edge of the broad peak. Due to the partially overlapping nature of the HBsAg-containing peak, care was taken to avoid collecting fractions in the trailing edge of the first peak and the leading edge of the second peak, even if they contain HBsAg molecules.

The HBsAg fractions from the gel filtration column chromatography were pooled and processed in the next step by KBr isopycnic ultracentrifucation. The pooled fractions amount to 150-200 ml processed from the 5L cell culture medium. Solid KBr was then added to the pooled fractions containing HBsAg to make the final concentration of KBr at 24-26% (w/w). The sample solution was divided into two Quick-Seal tubes (Beckman, 100 ml capacity each) and centrifuged at 45,000 rpm for 20-24 hrs. at 15 degree C using a Beckman rotor, Ti45.

A linear gradient of KBr formed as a result of the ultracentrifugation was fractionated using a gradient fractionation system (Isco) and a UV monitor. A gradient fractionation profile monitored at 280 nm showed three peaks well-separated from each other. Refractive index measurements and SDS-PAGE analysis indicated that the broad middle peak contained a nearly pure population of HBsAg particles banded by the KBr ultracentrifugation around 1.2 g/ml. The particle purity of the combined middle peak fractions as measured by SDS-PAGE under non-reducing conditions reached more than 95%, although the protein purity appears to be 85-90% as determined by appropriate current techniques such as gel scanning and immunoblot staining.

To proceed into a sucrose step gradient ultracentrifugation, about 50 ml of the pooled HBsAg fraction was subjected to a concentration-dialysis with a stirred cell (Amicon) using a XM-300 membrane at a pressure of 20 psi. After the initial concentration, PBS was added 3-5 times followed by concentration each time to effectively remove KBr contained in the HBsAg fraction. About 10 ml of concentrated HBsAg solution was obtained as a result of this pressure dialysis operation.

The concentrated HBsAg fraction was then loaded on a sucrose step-gradient consisting of 20%, 30% and 40% (w/w). 10 ml each of the above mentioned sucrose steps was layered in a 35 ml capacity centrifuge tube (Beckman), and 5 ml of the sample solution was loaded on top of the gradient. Ultracentrifugation was carried out at 4~C using Beckman rotor SW-28 at 25000 rpm for 16 hr. A gradient fractionation profile monitored at 280 nm after the sucrose step gradient ultracentrifugation showed a major single peak followed by a well-separated minor peak. This minor peak was shown by SDS-PAGE and silver staining to contain high molecular weight contaminants in excess of 66,000 daltons.

The final product from the sucrose step gradient centrifugation was subjected to dialysis and/or concentration using an Amicon stirred cell with XM-300 membrane. Microscopic examination indicated the presence of roughly spherically particles about 22 nm in diameter. The concentrated solution was filtered through a 22 micron filter, lyophilized and stored at 4~C until used.

The yield and recovery through the 5L process is as shown in Table 1. The purified HBsAg product is greater than 98% pure.

Table 2

| Yield and recovery in 5L process | | | | |
|---|---|---|---|---|
| | total volume(ml) | HBsAg conc (ug/ml) | total HBsAg (mg) | % yield |
| 10 fold concentration | 500 | 55.45 | 27.7 | 100 |
| PEG precipitation | 26 | 918.75 | 23.9 | 86 |
| AcA 22 column chromatography | 304 | 66.7 | 20.3 | 73 |
| KBr isopycnic ultracentrifugation | 15 | 862.25 | 12.9 | 47 |
| Sucrose density ultracentrifugation | 19 | 489.5 | 9.3 | 33 |

EXAMPLE 9

To purify HBsAg from human plasma, a two-step procedure was employed in which a certain quantity of proteins including fibrinogen was removed in the first precipitation step and then a maximum amount of HBsAg was recovered in the second stage. Such sequential precipitation is not required in the purification of HBsAg from the cell culture medium, because of the differences in composition between culture medium and human plasma. The protein precipitates obtained by a two-step procedure (using 3% followed by 12% PEG precipitation) or by a one-step procedure (10% PEG precipitation) were subjected to gel filtration column chromatography according to the present invention. The fractions containing HBsAg were compared in terms of the quantity and composition of impurities and yield of HBsAg. There was essentially no difference between these two preparations, and therefore, the simpler one step precipitation is preferred.

EXAMPLE 10

A purified HBsAg product was prepared as in Example 2. The relative amounts of the protein components of the HBsAg were then determined. A sample of the HBsAg was separated by sodium dodecyl sulfate polyacrylamide gel electrophoresis. The gel was then treated by the silver-stain method of Merril. One track of the stained gel was then subjected to a densitometry trace. The densitometer measures the transmission of light through the bands of the gel, and indicates the staining intensity. The results are presented in Table 3.

Table 3

| Desitometry trace data for silver stained sample of purified HBsAg after SDS-PAGE | | | | |
|---|---|---|---|---|
| PEAK | LOCATION | PEAK MAX | PEAK AREA | % TOTAL AREA |
| A (dye front) | 6.86 | 0.6430 | 0.4318 | 1.29 |
| B (S: p24) | 20.07 | 2.8597 | 13.4299 | 40.19 |
| C (S: gp27) | 27.18 | 2.9370 | 12.9122 | 38.64 |
| D (pre-S2) | 38.35 | 1.2407 | 4.4275 | 13.25 |
| E (pre-S1: gp41) | 47.24 | 0.6558 | 1.0078 | 3.02 |
| F (pre-S1: gp44) | 49.53 | 0.5402 | 1.2070 | 3.61 |

The pre-S2 components in this example did not resolve into 2 separate peaks, but rather were seen as a peak with a shoulder. The total stain density of each S, pre-S1, and pre-S2 component can vary from sample to sample. Furthermore, it was observed that the density of silver staining was not proportional to the density of staining by Coomassie Blue, suggesting that the quantities presented in Table 2 are not stoichiometric. Rather, the intensity of silver-staining probably depends on the composition of the protein, including the degree of glycosylation. The relative staining intensity is, however, reproducible for a given protein mixture, and can thus be used to quantify the components of a protein mixture such as the heterogeneous HBsAg of this invention.

**Claims**

1. A method for purifying heterogeneous hepatitis B surface antigen, comprising isolating a crude preparation of heterogeneous HBsAg particles from a mammalian cell culture medium; then purifying heterogeneous pre-S rich HBsAg comprising substantial amounts of S, pre-S1 and pre-S2 components as determined by gel electrophoresis, wherein the purifying step is free of a process that acts by separating HBsAg particles on the basis of charge or chemical affinity, and employs a process that separates the particles on the basis of size separation or density separation.

2. The method of claim 1, wherein the isolating step comprises:

(a) obtaining a mammalian cell culture medium containing heterogeneous HBsAg particles; then

(b) fractionally precipitating heterogeneous HBsAg particles from the medium; and the purifying step comprises:

(c) separating a fraction of heterogeneous HBsAg particles by gel filtration chromatography, then

(d) separating heterogeneous HBsAg particles by ultracentrifugation.

3. The method of claim 2, in which step (d) comprises isopycnic banding ultracentrifugation and sucrose step gradient ultracentrifugation.

4. The method of claim 2 wherein step (a) further comprises clarifying and concentrating the cell culture medium about 10 to 50 fold.

5. The method of claim 4 wherein the clarifying is by ultracentrifugation and the concentration is by ultrafiltration.

6. The method of claim 5 wherein the ultrafiltration uses a filter having a nominal molecular weight cut off less than about 300,000 daltons.

7. The method of claim 1 wherein the cell culture medium contains up to about 25% serum.

8. The method of claim 2 wherein the fractional precipitation in step (b) is a single step process using about 9%-12% polyethylene glycol 6000-9000.

14

9. The method of claim 2 wherein the gel filtration column chromatography employs a medium having molecular sieving properties capable of separating HBsAg particles from higher and lower molecular weight components.

10. The method of claim 3 wherein the isopycnic banding ultracentrifugation uses about 24%-26% (w/w) potassium bromide to form a density gradient.

11. The method of claim 3 wherein the sucrose step gradient includes steps containing about 20%, about 30% and about 40% sucrose (w/w).

12. The method of claim 2 further comprising the step of lyophilizing the purified HBsAg product.

13. The method of claim 2, further comprising the step of heat inactivation and potentiation of the purified HBsAg product.

14. The method of claim 2, further comprising the step of blending the purified HBsAg product with adjuvant to produce a vaccine.

15. A recombinant DNA expression vector comprising a gene determinant for HBsAg comprising pre-S1, pre-S2, and S region DNA from HBV and an inducible control region, inducible by an inducer, a gene conferring antibiotic resistance to a bacterial host, and a gene conferring selective advantage on transfected cells relative to untransformed cells in the presence of a selecting agent, the HBsAg gene being positioned relative to the control region such that it is subjected to the control of the control region, whereby upon introduction of the vector into a bacterial host, the vector can be cloned by selection with the antibiotic, and whereby upon introduction of the vector into a mammalian cell line host, transfected cells can be selected in the presence of the selective agent and the control region is able to allow production of pre-S rich HBsAg to proceed in response to presence of the inducer, and the mammalian cell line host is capable of secreting heterogenecus, pre-S rich HBsAg particles.

16. Plasmid pHPS1.

17. The vector according to claim 15, further comprising a transformer gene capable of transforming a mammalian cell line.

18. The vector of claim 17, wherein the control region is a mouse metallothionein gene DNA sequence inducible by cadmium or zinc, the gene conferring resistance confers resistance to ampicillin, the gene conferring selective advantage is a metallothionein gene, and the transformer gene is bovine papilloma virus.

19. A mammalian cell line transfected by the vector of claim 15.

20. A mouse fibroblast cell line transfected and transformed by the vector of claim 18.

21. A method of producing pre-S rich, heterogeneous HBsAg particles comprising:

(a) transfecting mammalian cell line cells with the vector of claim 15;

(b) selecting transfected cells with a selecting agent; and

(c) inducing expression of HBsAg with an inducer, whereby the cell line secretes heterogeneous, pre-S rich HBsAg particles.

## FIGURE 1

HBsAg SECRETING
MAMMALIAN CELL CULTURE

1A:  Clarification

1B:  Concentration

CLARIFIED CULTURE MEDIUM

2:  Fractional
    Precipitation

HBsAg PELLET

3:  Gel Filtration
    Chromatography

HBsAg FRACTIONS

4:  Isopycnic Banding
    Ultracentrifugation

HBsAg FRACTIONS

5:  Sucrose Step
    Gradient
    Ultracentrifugation

PURE HETEROGENEOUS HBsAg PARTICLES

FIGURE 2

FIGURE 3

FIGURE 4

FIGURE 5

FIGURE 6

FIGURE 7

BamH1

Hha1

pHB1          1.35

Hha1

BamH1

pCS2

Xho1

Hha1          Xho1  (there is only
                            one Xho1 site)

1345b DNA   (there are many       BAP
                  Hha1 fragments)

MIX

T4 polymerase

Ligase

16.5Kb

pHPS7
17.8Kb

1.35

FIGURE 8

• | lost Xho1 sites

FIGURE 9

FIGURE 10

FIGURE 11

FIGURE 12

FIGURE 13

FIGURE 14

FIGURE 15

FIGURE 16

FIGURE 17

FIGURE 18

FIGURE 19

European Patent Office

**EUROPEAN SEARCH REPORT**

Application number

## DOCUMENTS CONSIDERED TO BE RELEVANT

EP 89102269.1

| Category | Citation of document with indication, where appropriate, of relevant passages | Relevant to claim | CLASSIFICATION OF THE APPLICATION (Int. Cl.4) |
|---|---|---|---|
| X | EP - A1 - 0 198 474 (ENDOTRONICS INC.)<br>* Claims 1,3,10 * | 15,18, 19 | A 61 K 39/29<br>A 61 K 39/40<br>C 12 N 15/00 |
| A | EP - A2 - 0 243 913 (CALIFORNIA INSTITUTE OF TECHNOLOGY)<br>* Abstract; claims * | 21 | |
| A | WO - A1 - 86/05 189 (SCRIPPS CLINIC AND RESEARCH FOUNDATION)<br>* Claims 1,28 * | 21 | |
| A | WO - A1 - 85/03 876 (GROUPEMENT DE GENIE GENETIQUE)<br>* Claims 7,17,19 * | 15,17-21 | |
| D,A | US - A - 4 683 136 (MILICH et al.)<br>* Abstract; claims * | 21 | TECHNICAL FIELDS SEARCHED (Int. Cl.4)<br><br>A 61 K 39/00<br>C 12 N 15/00 |
| A | EP - A1 - 0 244 924 (MERCK & CO. INC.)<br>* Claims 1-4 * | 15 | |
| P,A | EP - A1 - 0 280 470 (MERCK & CO. INC.)<br>* Claim 1 * | 1 | |

The present search report has been drawn up for all claims

| Place of search | Date of completion of the search | Examiner |
|---|---|---|
| VIENNA | 08-05-1989 | SCHNASS |